(19) **Europäisches Patentamt European Patent Office Office européen des brevets**

(11) **EP 0 993 834 B1**

(12) **EUROPEAN PATENT SPECIFICATION**

(45) Date of publication and mention of the grant of the patent:
**27.06.2007 Bulletin 2007/26**

(51) Int Cl.:
**B01J 20/32** [(2006.01)]

(21) Application number: **98912798.0**

(22) Date of filing: **13.04.1998**

(86) International application number:
**PCT/JP1998/001704**

(87) International publication number:
**WO 1998/047548 (29.10.1998 Gazette 1998/43)**

(54) **ADSORBENTS FOR TOXIC SHOCK SYNDROME TOXIN-1, METHOD FOR ELIMINATING THE TOXIN BY ADSORPTION**

ADSORBENTIEN FÜR TOXISCHES SCHOCKSYNDROM TOXIN-1, VERFAHREN ZUR BESEITIGUNG DES TOXINS DURCH ADSORBTION

ADSORBANTS DE LA TOXINE-1 DU SYNDROME DE CHOC TOXIQUE, PROCEDE D'ELIMINATION DE LADITE TOXINE PAR ADSORPTION

(84) Designated Contracting States:
**DE FR GB IT**

(30) Priority: **21.04.1997 JP 10357697**

(43) Date of publication of application:
**19.04.2000 Bulletin 2000/16**

(73) Proprietor: **KANEKA CORPORATION Osaka 530-8288 (JP)**

(72) Inventors:
• **HIRAI, Fumiyasu Hyogo 661-0047 (JP)**
• **OGINO, Eiji Kobe-shi,Hyogo 650-0004 (JP)**
• **MARUYAMA, Hiroyuki Kakogawa-shi,Hyogo 675-0051 (JP)**
• **SAKOGAWA, Takayuki Hyogo 676-0025 (JP)**
• **ASAHI, Takashi Kobe-shi,Hyogo 655-0853 (JP)**

• **TANI, Nobutaka Osaka-shi,Osaka 545-0004 (JP)**

(74) Representative: **Vossius & Partner Siebertstrasse 4 81675 München (DE)**

(56) References cited:
**EP-A- 0 247 592        EP-A- 0 723 794
EP-A- 0 743 067        JP-A- 5 253 479**

• **SCHLIEVERT P.M. et al., "Identification and Characterization of an Exotoxin from Stapylococcus Aureus Associated with Toxic-Shock Syndrome", THE JOURNAL OF INFECTION DISEASES, Vol. 143, No. 4, (1981), p. 509-516, XP002911213**
• **IGARASHI H. et al., "Purification and Characterization of Stapylococcus Aureus FRI1169 and 587 Toxic Shock Syndrome Exotoxins", INFECTION AND IMMUNITY, Vol. 44, No. 1, (1984), p. 175-181, XP002911214**

**Description**

The present invention relates to adsorption of toxin shock syndrome toxin-1.

The toxic shock syndrome toxin-1 (hereinafter referred to as "TSST-1") is an exotoxin composed of a soluble protein having a molecular weight of about 20-30 kDa produced by Staphylococcus aureus, and is a representative superantigen.

Sepsis refers to a state that an infection exists somewhere in a body, whereby a systemic inflammatory response has occurred. If this inflammatory symptom accelerates, a shock symptom (septic shock) occurs, and organopathy (organ failure) also occurs, further falling into such a gave state as multi-organ failures. The source of that infection is mainly bacteria, and the bacteria is roughly classified into Gram-positive bacteria and Gram-negative bacteria.

If infected with Staphylococcus aureus which is a sort of Gram-positive bacteria, TSST-1 produced by the infecting Staphylococcus aureus propagates and activates T-cells to cause sepsis. Recently, infection with methicillin resistant Staphylococcus aureus (MRSA) and septic shock accompanied thereby attract attention as an important prophlogistic bacteria of nosocomial infection and have come a large social problem for the last several years (Tomoyuki Kawamata et al., Intensive & Critical Care Medicine, Vol. 7, page 631, 1995).

On the other hand, if infected with Gram-negative bacteria, endotoxin present in the cell wall of the bacteria enters into blood to cause sepsis. Further, in recent years, it is reported that since TSST-1 activates immune system as a superantigen to enhance the toxicity of endotoxin to several thousands times, sepsis is caused even by the presence of such a low concentration of endotoxin that cannot clinically cause sepsis. Thus, in case of mixed infection with both Gram-positive bacteria and Gram-negative bacteria, a possibility of causing sepsis becomes very high.

Antibiotics as a countermeasure for infection and $\gamma$-globulin to activate the resistance to infection have been used for the treatment of sepsis, but the mortality is still high. For the reason, it has been desired from the medical point of view to remove TSST-1 and endotoxin which become a cause of sepsis from body fluids.

As to endotoxin, adsorbents to remove it from body fluids are known. For example, Japanese Patent Publication Kokoku No. 1-16389 discloses an adsorbent wherein polymyxin known as an antidote to endotoxin is immobilized on a suitable carrier. Also, the present inventors disclose in Japanese Patent Publication Kokai No. 8-173803 that endotoxin can be adsorbed by a sulfo group-introduced styrene-divinyl benzene copolymer. These adsorbents are expected to produce a fairly large effect against infection with Gram-negative bacteria. However, in case that both TSST-1 and endotoxin coexist as a result of mixed infection with Gram-positive bacteria and Gram-negative bacteria, the effect lowers. Further, in case that a person is infected with only Gram-positive bacteria and TSST-1 enters a body fluid, no effect is expected. Various adsorbents for endotoxin are known, but no adsorbent for TSST-1 has been known. Thus, development of adsorbent for TSST-1 has been strongly desired.

EP-A-0743067 relates to a material for detoxification or elimination of super antigens such as staphylococcal enterotoxin and streptococcal exotoxin.

An object of the present invention is to solve the problem of efficiently removing TSST-1 present in body fluids.

DISCLOSURE OF INVENTION

The invention relates to the use of an adsorbent material for the manufacture of an adsorptive medicament as defined in claim 1.

The adsorbent mentioned in claim 1, is immobilized on a water-insoluble porous carrier.

The water-insoluble carrier has an exclusion limit for globular protein of from $1 \times 10^4$ to $60 \times 10^4$.

Further, the present invention relates to (4) a method for removing TSST-1 from a body fluid in a bag characterized by bringing a body fluid containing TSST-1 into contact with the adsorbent material as defined in claim 1.

Fig. 1 is a schematic cross section view showing an example of an adsorber for TSST-1 and
Fig. 2 is a graph showing results of examining a relationship between the flow rate and the pressure drop $\Delta P$ by using three kinds of water-insoluble carriers.

The "TSST- 1" in the present invention is an exotoxin composed of a soluble protein having a molecular weight of about 20-30 kDa produced by Staphylococcus aureus.

Also, the body fluid means blood, plasma, serum, ascites, lymph, synovia and fractions obtained from them, and other liquid components derived from a living body.

The log P value is a parameter which indicates the hydrophobicity of a compound. A typical partition coefficient P in an octanol-water system is determined as follows: At first, a compound is dissolved in octanol (or water) and an equal amount of water (or octanol) is added thereto. After shaking the mixture for 30 minutes with a Griffin flask shaker (made by Griffin & George Ltd.), the mixture is centrifuged for 1 to 2 hours at 2,000 r.p.m. The respective concentrations of the compound in both octanol and water layers are measured by various methods such as spectroscopic method or GLC, and the partition coefficient P is obtained from the following equation.

$$P = Coct/Cw$$

Coct: concentration of a compound in the octanol layer
Cw: concentration of a compound in the water layer

The adsorbent used in the present invention comprises a compound selected from n-octylamin, cetylamin or cetyl bromide.

Until now, many investigators have determined log P values of various compounds and the found values of the log P are put in order by C. Hansch et al ("PARTITION COEFFICIENTS AND THEIR USES"; Chemical Reviews, 71, page 525 (1971)).

As to compounds whose found values are unknown, calculated values (Σf) obtained by using a hydrophobic fragmental constant f shown in R.F. Rekker's book ("THE HYDROPHOBIC FRAGMENTAL CONSTANT", Elsevier Sci. Pub. Com., Amsterdam, 1977) can be a good guide. The hydrophobic fragmental constant f is a value showing the hydrophobicity of various fragments determined by a statistical treatment of many found values of log P. The sum of f values of respective fragments which constitute a compound approximately agree with log P of the compound. In the present disclosure, the log P value of a compound means Σf value when the log P value of the compound is not known.

In investigating compounds effective for adsorbing TSST-1, compounds having various log P values were immobilized on a water-insoluble carrier and the adsorption ability thereof were examined with respect to TSST-1. As a result, it has been found that compounds having a log P value of 2.50 or more, preferably 2.80 or more, more preferably 3.00 or more, are effective for adsorption of TSST-1, and that compounds having a log P value of less than 2.5 hardly show an adsorption ability for TSST-1. For example, in the case of immobilizing an alkylamine on a water-insoluble carrier, it has been found that the absorption ability for TSST-1 is enhanced to a great extent when the alkylamine is changed from n-hexylamine (log P = 2.06)) to n-octylamine (log P = 2.90). From these results, it is assumed that the adsorption ability of the adsorbent according to the present invention with respect to TSST-1 is based on a hydrophobic interaction between TSST-1 and an atomic group introduced onto a carrier by immobilization of a compound having a log p value of 2.50 or more, and that a compound having a log P value of less than 2.50 does not show any ability for adsorbing TSST-1 because the hydrophobicity of this compound is too low.

The "water-insoluble carrier" in the adsorbent of the present invention means a carrier which is solid at ordinary temperature under ordinary pressure and is insoluble in water.

The water-insoluble carrier in the present invention may be in the form of, for example, particle, board, fiber, hollow fiber, and the like, but the form thereof is not limited thereto. The size of the carrier is not also particularly limited.

Typical examples of the water-insoluble carrier in the present invention are, for instance, inorganic carriers such as glass beads and silica gel, organic carriers each comprising synthetic polymers such as crosslinked or non-crosslinked polyvinyl alcohol, crosslinked or non-crosslinked polyacrylate, crosslinked or non-crosslinked polyacrylamide and crosslinked or non-crosslinked polystyrene, or polysaccharides such as crystalline celluloses, crosslinked or non-crosslinked celluloses, crosslinked or non-crosslinked agarose and crosslinked or non-crosslinked dextrin, and composite carriers each comprising a combination of the above-mentioned materials such as organic-organic carriers and organic-inorganic carriers.

Among these carriers, hydrophilic carriers are preferable since non-specific adsorption is comparatively a little and the adsorption selectivity for TSST-1 is good. The term "hydrophilic carrier" as herein used refers to a carrier composed of a material which has a contact angle with water of 60 degrees or less when the material is shaped into a flat plate. Typical examples of such hydrophilic carriers are, for instance, those comprising cellulose, polyvinyl alcohol, hydrolyzed ethylene-vinyl acetate copolymer, polyacrylamide, polyacrylic acid, polymethacrylic acid, polymethyl methacrylate, polyacrylic acid-grafting polyethylene, polyacrylamide-grafting polyethylene, glass, and the like.

Of these, a porous cellulose gel is one of the most suitable carriers, since it has the superior properties that (1) the gel is hard to be destroyed or to become fine powder by an operation such as agitation because it has a comparatively high mechanical strength and toughness, so when the gel is packed in a column, compaction and choking of the column do not occur even if a body fluid is flowed at a high flow rate, and further the porous structure of the gel is hard to receive a change by a high pressure steam sterilization or the like, (2) since the gel is made of a cellulose, the gel is hydrophilic, and many hydroxyl group which can be utilized for bonding a ligand are present, and non-specific adsorption hardly occurs, (3) the adsorption capacity which is comparable to a soft gel can be obtained because the gel has a comparatively high strength even if the pore volume is made large, and (4) the safety is higher than synthetic polymer gels and the like. The carrier used in the present invention is preferably a hard gel that the flow rate does not increase in proportion to increase of the pressure, but is not limited thereto. The above-mentioned carriers may be used alone or in admixture thereof.

The property required for the water-insoluble carrier used in the present invention is to have a large number of pores having an adequate size, namely to be porous. TSST-1 which is the subject of adsorption by the adsorbent of the present invention is a protein having a molecular weight of about 20-30 kDa. It is preferable for efficiently adsorbing this protein that TSST-1 can enter the fine pores at some large probability but other proteins enter the pores as little as possible. Exclusion limit has been generally used as a measure of the molecular weight of a substance which can enter fine pores. The term "exclusion limit" means the molecular weight of the smallest molecule of molecules which cannot enter fine pores (namely which are excluded) in a gel permeation chromatography, as described in books (see, for example, Hiroyuki Hatano and Toshihiko Hanai, "Experimental High Performance Liquid Chromatography", Kagaku Dojin). In general, the exclusion limit has been well examined with use of globular protein, dextran, polyethlene glycol or the like. In the case of the water-insoluble porous carrier used in the present invention, it is suitable to use the values obtained by using globular protein.

As a result of study using carriers of various exclusion limits, it has become clear that the range of exclusion limit suitable for adsorbing TSST-1 is from $1 \times 10^4$ to $60 \times 10^4$. That is to say, when a carrier having an exclusion limit of less than $1 \times 10^4$ is used, the amount of TSST-1 adsorbed is small, so the practicability becomes low. When a carrier having an exclusion limit of more than $60 \times 10^4$ is used, proteins (mainly albumin) other than TSST-1 are adsorbed in an increased amount, so the practicability is low from the viewpoint of selectivity. Accordingly, the exclusion limit of the carrier used in the present invention is from $1 \times 10^4$ to $60 \times 10^4$, preferably from $1.5 \times 10^4$ to $40 \times 10^4$, more preferably from $2 \times 10^4$ to $30 \times 10^4$.

As to the pore structure of carriers, it is preferable from the viewpoint of the adsorption ability per unit volume of an adsorbent that the carrier is porous throughout the entire thereof rather than being porous only in the surface region. Preferably, the pore volume is at least 20 % and the specific surface area is at least 3 $m^2/g$.

Further it is preferable that the carrier has a functional group which can be used in a reaction for immobilizing a ligand to the carrier. Typical examples of the functional group are, for instance, hydroxyl group, amino group, aldehyde group, carboxyl group, thiol group, silanol group, amide group, epoxy group, halogen, succinimide group, acid anhydride group, and the like. The functional groups are not limited to the exemplified groups.

Any of a hard carrier and a soft carrier can be used as the water-insoluble carrier in the present invention. In the case of using an adsorbent for an extracorporeal circulation, it is important that the adsorbent does not clog up when it is charged in a column and a fluid is flowed through the column. For this purpose, a sufficient mechanical strength is required for the adsorbent. Accordingly it is more preferable to use a hard carrier as the water-insoluble carrier in the present invention. The term "hard carrier" as used herein refers to, for example, in the case of a granulated gel, a carrier which has such a property that a linear relation between pressure drop $\Delta P$ and flow rate is held up to a pressure drop of 0.3 $kg/cm^2$ when the gel is uniformly charged in a cylindrical column and an aqueous fluid is passed through it, as shown in Reference Example described after.

The adsorbent of the present invention is obtained by immobilizing a compound selected from n-octylamine, cetylamine or cetyl bromide. Various known methods of immobilization can be used without particular restriction. However, when the adsorbent of the present invention is used for an extracorporeal circulation treatment, it is important from the viewpoint of safety to suppress the elimination or elution of a ligand as much as possible in sterilization or treatment. For this purpose, preferably the immobilization is conducted by a covalent bond method.

In the adsorbent of the present invention, it is preferable that a proper amount of a compound selected from n-octylamine, cetylamine or cetyl bromide is immobilized. If the amount of the compound immobilized is too small, TSST-1 is not adsorbed. If the amount is too large, a sticky component such as platelets may adhere to the adsorbent when blood is used as a body fluid. Thus, the amount of the compound to be immobilized is preferably from 0.1 to 10,000 μmol, more preferably from 1 to 200 μmol, the most preferably from 5 to 100 μmol, per unit volume (1 ml) of a water-insoluble carrier.

Various methods are adoptable for adsorbing and removing TSST-1 from a body fluid by using the adsorbent of the present invention. The most simple method is a method wherein a body fluid is taken out and placed in a bag or the like and the adsorbent is mixed therewith to allow to adsorb TSST-1 and then the adsorbent is filtered off to obtain the body fluid from which TSST- 1 has been removed. Another method which is not part of the present invention is a method wherein the adsorbent is packed in a container which has an inlet and an outlet for a body fluid and which is equipped at least at the outlet with a filter which can pass a body fluid but cannot pass the adsorbent, and the body fluid is passed through the container. Both methods can be used, but the latter method is adequate for the adsorbent of the present invention, since the operation is simple and TSST-1 can be removed efficiently in on-line system from a body fluid, especially blood, of a patient by incorporating the method into an extracorporeal circulation circuit. In the extracorporeal circulation circuit, the adsorbent of the present invention can be used not only singly but also in combination with other extracorporeal circulation therapy systems. As an example of the combination use is mentioned, for instance, an artificial dialysis circuit, and the adsorbent can be used in a combination with dialysis therapy.

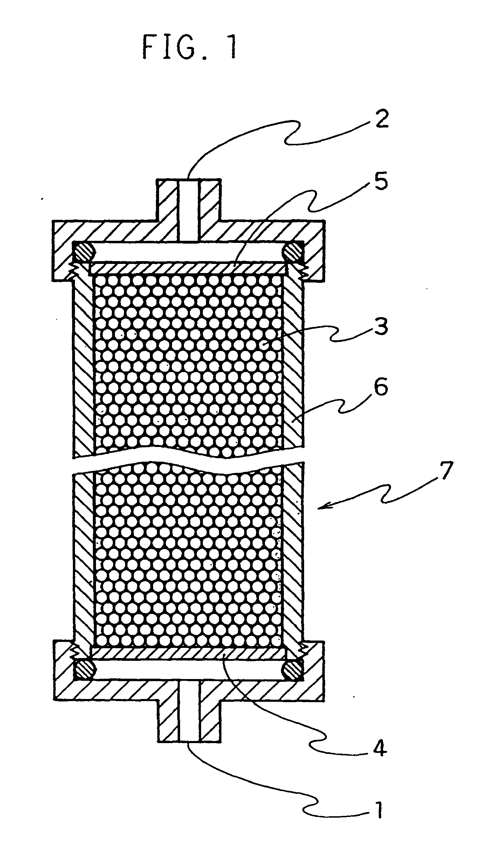

An adsorber for TSST-1 using the TSST-1 adsorbent mentioned above will be explained below with reference to Fig. 1 which is a schematic section view showing an example of the adsorber. In Fig. 1, 1 denotes an inlet for a body fluid, 2 denotes an outlet for the body fluid, 3 denotes the TSST-1 adsorbent, 4 and 5 denote a filter (filter for preventing the adsorbent from flowing out) which can pass a body fluid and components included therein but cannot pass the adsorbent, 6 denotes a column, and 7 denotes an adsorber for TSST-1. The TSST-1 adsorber is not limited to such an exemplified adsorber, and any devices can be adapted so long as the devices have a structure that the adsorbent mentioned before is packed in a container having an inlet and an outlet for a body fluid and equipped with a means for preventing the adsorbent from flowing out of the container.

Examples of the means for preventing the adsorbent from flowing out are, for instance, filters such as mesh, nonwoven fabric and cotton stopper. There is no particular limitation in the shape, material and size of the container, but a preferable example of the container is a transparent or semitransparent cylindrical container which, for instance, has a capacity of about 150 to about 400 ml and a diameter of about 4 to about 10 cm. Particularly preferable materials are those having a sterilization-resistance. Typical examples of such materials are, for instance, glass coated with a silicone, polypropylene, polyvinyl chloride, polycarbonate, polysulfone, polymethylpentene, and the like.

The present invention is explained below in more detail by means of Examples.

REFERENCE EXAMPLE

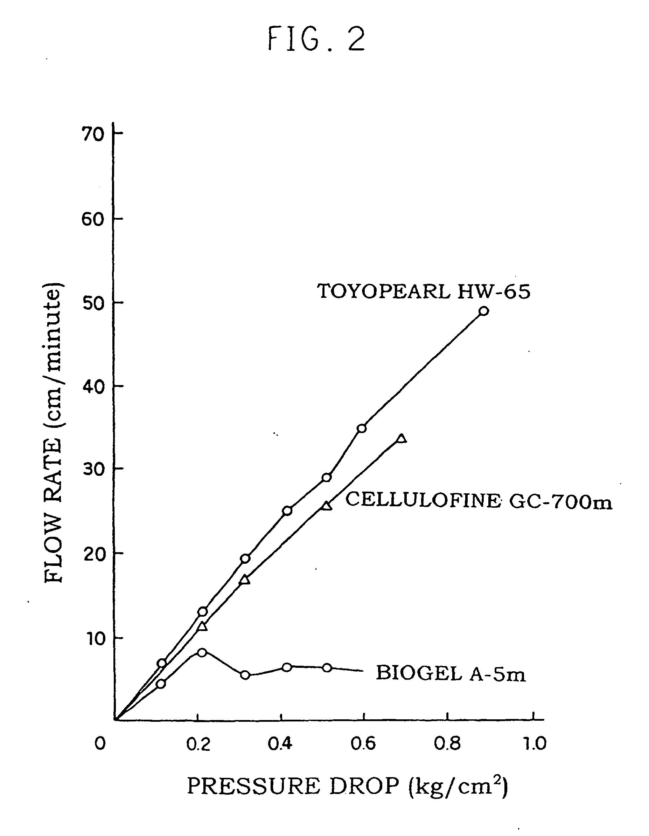

Cylindrical glass columns (inner diameter 9 mm, length 150 mm) equipped with filters having a pore size of 15 $\mu$m at both ends, were charged uniformly with each of an agarose gel (Biogel A-5m made by Bio-Rad Laboratories, U.S.A., particle size 50 to 100 meshes), a vinyl polymer gel (TOYOPEARL HW-65 made by TOSOH Corporation, Japan, particle size 50 to 100 $\mu$m) and a cellulose gel (CELLULOFINE GC-700m made by Chisso Corporation, Japan, particle size 45 to 105 $\mu$m). The relationship between flow rate and pressure drop $\Delta$P was determined by passing water through the column with a peristatic pump. The results are shown in Fig. 2.

In Fig. 2, it is found that TOYOPEARL HW-65 and CELLULOFINE GC-700m show that the flow rate increases almost in proportion to an increase in pressure, whereas Biogel A-5m causes a compaction and the flow rate does not increase even if the pressure is increased. In the present invention, a gel showing that the relationship between the pressure drop $\Delta$P and the flow rate is linear up to a pressure drop of 0.3 kg/cm$^2$, like the former, is referred to as a hard gel.

EXAMPLE 1

Water was added to 170 ml of a porous cellulose gel CELLULOFINE GC-200m (made by Chisso Corporation, Japan, exclusion limit for a globular protein 140,000) up to the total amount of 340 ml, and thereto was added 90 ml of a 2M aqueous solution of sodium hydroxide, and the mixture was kept at 40°C. To the mixture was then added 31 ml of epichlorohydrin, and the reaction was carried out with stirring at 40°C for 2 hours. After the completion of the reaction, the gel was thoroughly washed with water to give an epoxidized gel.

To 10 ml of the epoxidized gel was added 200 mg of n-octylamine (log P = 2.90), and the mixture was allowed to stand for reaction in a 50 v/v % aqueous solution of ethanol at 45°C for 6 days to immobilize. After the completion of the reaction, the gel was thoroughly washed with a 50 v/v % aqueous ethanol solution, ethanol, a 50 v/v % aqueous ethanol solution and water in that order to give a n-octylamine-immobilized gel.

To 0.2 ml of the immobilized gel (adsorbent) was added 1.2 ml of a TSST-1-containing serum (TSST-1 concentration 5 ng/ml) prepared by adding TSST-1 (made by Toxin Technology Corporation, U.S.A.) to a fetal calf serum (made by Flow Laboratories, Australia). The resulting mixture was incubated at 37°C for 2 hours. The same procedure was conducted also with respect to 0.2 ml of physiological saline used instead of the adsorbent. Concentrations of TSST-1 in the supernatant before and after the incubation were measured by an ELISA method, and the adsorption rate was calculated by dividing the concentration in the supernatant obtained with the use of the adsorbent by the concentration in the supernatant obtained with the use of physiological saline (percentage).

The ELISA method for TSST-1 was conducted as follows: A primary antibody Rabbit Anti-TSST- 1 IgG (made by Toxin Technology Corporation, U.S.A.) was diluted with a coating buffer to 1,600 times, and 100 $\mu$l portions thereof were distributed onto a microplate. After allowing to stand overnight at 4°C, the microplate was washed, and 200 $\mu$l portions of a 3 % solution of bovine serum albumin were distributed onto the microplate. After allowing to stand at room temperature for 2 hours, the microplate was washed, and 100 $\mu$l of each of a TSST-1 standard liquid and the supernatants before and after incubation was added to the microplate. After allowing to stand at room temperature for 2 hours, the microplate was washed. A secondary antibody Rabbit Anti-TSST-1 HRPO (made by Toxin Technology Corporation, U.S.A.) was diluted with a 1 % solution of bovine serum albumin to 400 times, and 100 $\mu$l portions thereof were distributed onto the microplate. After allowing to stand at room temperature for 2 hours, the microplate was washed. Thereto were distributed 100 $\mu$l portions of an o-phenylenediamine solution, and the microplate was allowed to stand at room temperature for 10 minutes. To the microplate were distributed 100 $\mu$l portions of 4N sulfuric acid and the absorbance was measured at 492 nm. The concentrations of TSST-1 in the supernatants before and after incubation was obtained by comparison with the absorbance of the standard liquid.

EXAMPLE 2

A cetylamine-immobilized gel was prepared in the same manner as in Example 1 except that cetylamine ($\Sigma f$ = 7.22) was used instead of n-octylamine (log P = 2.90) and ethanol was used as a solvent for the immobilization reaction. Adsorption test was made in the same manner as in Example 1 by using this adsorbent, and the concentration of TSST-1 was measured and the adsorption rate was calculated.

EXAMPLE 3

A cetylamine-immobilized gel was prepared in the same manner as in Example 1 except that CELLULOFINE GC-700m (made by Chisso Corporation, Japan, exclusion limit for a globular protein 400,000) was used instead of CELLULOFINE GC-200m. Adsorption test was made in the same manner as in Example 1 by using this adsorbent, and the concentration of TSST-1 was measured and the adsorption rate was calculated.

EXAMPLE 4

To 10 ml of CELLULOFINE GC-200m were added 10 ml of t-butyl alcohol and 2.0 g of potassium butoxide, and the resulting mixture was stirred at 40°C for 1 hour. Then 2.0 ml of cetyl bromide ($\Sigma f$ = 9.71 after immobilization) was added to the mixture and stirred for 4 hours. After the reaction, the gel was filtered off and washed with ethanol and water to give a cellulose gel onto which cetyl group is bound by ether bond. Adsorption test was made in the same manner as in Example 1 by using this adsorbent, and the concentration of TSST-1 was measured and the adsorption rate was calculated.

COMPARATIVE EXAMPLE 1

A n-butylamine-immobilized gel was prepared in the same manner as in Example 1 except that n-butylamine (log P = 0.97) was used instead of n-octylamine (log P = 2.90). Adsorption test was made in the same manner as in Example 1 by using this adsorbent, and the concentration of TSST-1 was measured and the adsorption rate was calculated.

COMPARATIVE EXAMPLE 2

A n-hexylamine-immobilized gel was prepared in the same manner as in Example 1 except that n-hexylamine (log P = 2.06) was used instead of n-octylamine (log P = 2.90). Adsorption test was made in the same manner as in Example 1 by using this adsorbent, and the concentration of TSST-1 was measured and the adsorption rate was calculated.

Log P values or $\Sigma f$ values of the compounds used in the Examples and Comparative Examples and the adsorption rate (%) calculated are shown in Table 1.

TABLE 1

|  | log P ($\Sigma f$) value | Adsorption rate (%) |
|---|---|---|
| Example 1 | 2.90 | 38 |
| Example 2 | 7.22 | 79 |
| Example 3 | 7.22 | 78 |
| Example 4 | 9.71 | 85 |
| Com. Ex. 1 | 0.97 | 4 |
| Com. Ex. 2 | 2.06 | 1 |

According to the present invention, TSST-1 in body fluids can be efficiently removed by using an adsorbent comprising a compound selected from n-octylamine, cetylamine or cetyl bromide.

**Claims**

1. Use of an adsorbent material comprising a water-insoluble carrier and a compound immobilized on said carrier, wherein said compound is selected from n-octylamine, cetylamine or cetyl bromide, and wherein said water-insoluble carrier is a water-insoluble porous carrier having an exclusion limit for globular protein of 10,000 to 600,000, for the manufacture of an adsorptive medicament for treating a bacterially infected patient by extracorporeal adsorptive removal of toxic shock syndrome toxin-1 from a body fluid.

2. The use according to claim 1, wherein the body fluid is taken from a patient infected with Gram-positive bacteria.

3. A method for removing toxic shock syndrome toxin-1 (TSST-1) from a body fluid in a bag, wherein an adsorbent material as defined in claim 1 is mixed with the body fluid to allow the TSST-1 to adsorb, and then the adsorbent material is filtered off to obtain a body fluid from which TSST has been removed.

**Patentansprüche**

1. Verwendung eines adsorbierenden Materials, umfassend einen wasserunlöslichen Träger und eine Verbindung, die auf dem Träger immobilisiert ist, wobei die Verbindung aus n-Octylamin, Cetylamin oder Cetylbromid ausgewählt ist, und wobei der wasserunlösliche Träger ein wasserunlöslicher poröser Träger mit einer Ausschlussgrenze für globuläre Proteine von 10.000 bis 600.000 ist, zur Herstellung eines adsorptiven Medikaments zur Behandlung eines mit Bakterien infizierten Patienten durch extrakorporale adsorptive Entfernung von toxischem Schocksyndrom Toxin-1 aus einer Körperflüssigkeit.

2. Verwendung nach Anspruch 1, wobei die Körperflüssigkeit einem Patienten entnommen wird, der mit Gram-positiven Bakterien infiziert ist.

3. Verfahren zur Entfernung von toxischem Schocksyndrom Toxin-1 (TSST-1) aus einer Körperflüssigkeit in einem Beutel, wobei ein adsorbierendes Material wie in Anspruch 1 definiert mit der Körperflüssigkeit gemischt wird, um die Adsorption von TSST-1 zu ermöglichen, und dann das adsorbierende Material abfiltriert wird, um eine Körperflüssigkeit zu erhalten, aus der TSST entfernt worden ist.

**Revendications**

1. Utilisation d'un matériau adsorbant comprenant un véhicule insoluble dans l'eau et un composé immobilisé sur ledit véhicule, dans laquelle ledit composé est choisi parmi la n-octylamine, la cétylamine ou le bromure de cétyle, et dans laquelle ledit véhicule insoluble dans l'eau est un véhicule poreux insoluble dans l'eau ayant une limite d'exclusion pour une protéine globulaire de 10 000 à 600 000, pour la fabrication d'un médicament adsorbant destiné au traitement d'un patient infecté par des bactéries par élimination adsorbante extracorporelle de la toxine 1 du syndrome de choc toxique d'un fluide corporel.

2. Utilisation selon la revendication 1, dans laquelle le fluide corporel est prélevé d'un patient infecté par des bactéries Gram-positives.

3. Procédé d'élimination de la toxine 1 du syndrome de choc toxique (TSST-1) d'un fluide corporel dans une poche, dans lequel un matériau adsorbant tel que défini dans la revendication 1 est mélangé avec le fluide corporel pour permettre à la TSST-1 d'être adsorbé, puis le matériau adsorbant est éliminé par filtration pour obtenir un fluide corporel duquel la TSST a été éliminée.

FIG. 1

# FIG. 2

TOYOPEARL HW-65

CELLULOFINE GC-700m

BIOGEL A-5m

FLOW RATE (cm/minute) vs PRESSURE DROP (kg/cm$^2$)

## REFERENCES CITED IN THE DESCRIPTION

**Patent documents cited in the description**

- JP 1016389 B
- JP 8173803 A
- EP 0743067 A

**Non-patent literature cited in the description**

- **TOMOYUKI KAWAMATA et al.** *Intensive & Critical Care Medicine,* 1995, vol. 7, 631
- **C. HANSCH et al.** PARTITION COEFFICIENTS AND THEIR USES. *Chemical Reviews,* 1971, vol. 71, 525
- THE HYDROPHOBIC FRAGMENTAL CONSTANT. **R.F.** Rekker's book. Elsevier Sci. Pub. Com, 1977